# EUROPÄISCHE PATENTSCHRIFT

(11) **EP 2 521 519 B1**
(45) Veröffentlichungstag und Bekanntmachung des Hinweises auf die Patenterteilung: **19.08.2015**
(21) Anmeldenummer: 10711838.2
(22) Anmeldetag: 11.03.2010
(51) Int. Cl.: A61F 9/008

(54) **OPHTHALMOLOGISCHES LASERSYSTEM**
OPHTHALMOLOGIC LASER SYSTEM
SYSTÈME LASER OPHTALMOLOGIQUE

(30) Priorität: 12.03.2009 DE 102009012873
(43) Veröffentlichungstag der Anmeldung: 14.11.2012
(73) Patentinhaber: Carl Zeiss Meditec AG, 07745 Jena (DE)
(72) Erfinder: DICK, Manfred, 07926 Gefell (DE); REICH, Matthias, 07749 Jena (DE); GREBNER, Dieter, 07751 Grosslöbichau (DE)
(74) Vertreter: Beck, Bernard
(86) Internationale Anmeldenummer: PCT/EP2010/001499
(87) Internationale Veröffentlichungsnummer: WO 2010/102804

(56) Entgegenhaltungen:
- EP-A1- 1 977 725
- WO-A1-01/35881
- WO-A2-03/059563
- WO-A2-2004/026198
- WO-A2-2007/084627
- DE-A1- 19 940 712
- US-A- 5 230 334

## Beschreibung

Die Erfindung betrifft ein ophthalmologisches Lasersystem zur photodisruptiven Bestrahlung okulären Gewebes, insbesondere einer Augenlinse oder einer Cornea, mit einem Laser, dessen Strahlung als Beleuchtungslicht über einen Beleuchtungsstrahlengang, der eine Scannereinheit und eine Fokussieroptik aufweist, in einem Untersuchungsbereich fokussierbar ist.

Die Akkommodation ist die Fähigkeit des Auges, ein in nahezu beliebiger Entfernung befindliches Objekt auf der Netzhaut scharf abzubilden. Die dabei erforderliche Einstellung des Brechwerts des optischen Systems erfolgt im wesentlichen durch eine elastische Verformung der Linse. Die maximal mögliche Brechkraftänderung wird als Akkommodationsbreite bezeichnet. Sie kann bis zu 16 dpt betragen. Durch eine Verhärtung und/oder Verdickung der Augenlinse mit zunehmendem Lebensalter verringert sich ihre Akkommodationsbreite. Dies wird als Presbyopie oder Alterssichtigkeit bezeichnet. Als presbyop wird eine Augenlinse typischerweise angesehen, wenn ihre Akkommodationsbreite unter 3 dpt absinkt. Die Presbyopie ist kein pathologischer Prozess, sondern eine natürliche Alterserscheinung, die etwa ab dem 40. Lebensjahr beginnt. Es ist heute im Grundsatz akzeptiert, dass eine Veränderung der Linsensubstanz mit einem kontinuierlichen Wachstum, d. h. eine Dickenzunahmen der Linse mit dem Lebensalter, die Linsenhärte ansteigen lässt und dadurch einen limitierenden Faktor der Akkommodation darstellt. Ursache für die veränderte Linsenelastizität ist vermutlich eine Sklerose des Nucleus und des Kortex der Augenlinse.

In der Ophthalmologie ist vorgeschlagen worden, eine erhärtete Linse durch geeignete Schnitte oder Blasenfelder mittels einer laserchirurgischen Therapie, insbesondere durch Photodisruption (engl. "laser-induced optical breakdown"; LIOB) oder andere Inzisionen, wieder in einen Zustand der besseren Verformbarkeit zu versetzen. Damit soll die Akkommodationsfähigkeit der Linse teilweise regeneriert werden. In EP 1 212 022 B1 und in US 2004/0199149 A1 ist eine solche Vorgehensweise unter Verwendung eines Femtosekunden-Lasers beschrieben. In WO 2005/070358 A1 sind beispielsweise Methoden zur Ausführung unterschiedlicher, zusammenhängender Schnittflächen zur Erzeugung von Gleitebenen beschrieben. Ein erweitertes ophthalmologisches Lasersystem für die Presbyopietherapie ist beispielsweise in WO 2008/017428 A2 offenbart. Es handelt sich um eine Navigationsvorrichtung zur optischen Analyse und Bearbeitung der inneren Struktur der Augenlinse. Die Navigationsvorrichtung ist mit einer konfokalen Detektionseinrichtung ausgerüstet. Sowohl für die Beleuchtung zur Analyse der inneren Struktur als auch für die Bearbeitung ist derselbe Laser vorgesehen. Für die Analyse wird das in der Augenlinse rückgestreute Laserlicht detektiert, um die Position, Geometrie und Struktur der Augenlinse zu ermitteln. Anhand der detektierten inneren Struktur und der individuellen geometrischen Form der Augenlinse werden die bei der Bearbeitung zu erzeugenden Schnittgeometrien bestimmt. Zu diesem Zweck wird ein Grundmodell an die detektierte individuelle Geometrie angepasst. Auch US 2007/0173794 beschreibt ein System zur Presbyopietherapie, wobei die Schnittstruktur an die innere Linsenstruktur angepasst wird.

Das Dokument WO 01/35881 offenbart ein ophthalmologisches Lasersystem, wobei Interferenz der mehreren Laserstrahlen zur Ablation benutzt wird. Ferner offenbart WO 03/059563 ein ophthalmologisches Lasersystem wo die Bestrahlungspunkte dreidimensional und nicht-äquidistant angeordnet werden können.

Neuerdings wird stark fokussierte Strahlung von Femtosekunden-Lasern auch im Rahmen einer intrastromalen Keratomileusis (LASIK) verwendet, um in der Cornea Schnitte zu führen (Femto-LASIK). Derartige Geräte werden auch als Lasermikrokeratome bezeichnet. Dabei wird im Fokus eine Photodisruption erzeugt, welche zu einer minimalen Blasenbildung im stromalen Gewebe führt. Setzt man mittels eines Scannersystems Fokuspunkt (engl, "spot") an Fokuspunkt, so kann man beliebige Schnitte (Perforationen) in die Cornea einbringen. Solche Schnitte werden nachfolgend als Laserschnitte bezeichnet. Es ist beispielsweise aus US 2006/0155265 A1 bekannt, den Flap mittels eines Femtosekunden-Lasersystems zu schneiden. Die Abtragung (Ablation) des für eine refraktive Korrektur erforderlichen stromalen Gewebes erfolgt dann konservativ mittels eines Excimerlasers, so dass auf eine mechanische Bearbeitung vollständig verzichtet werden kann. Aus WO 2008/064771 A1 ist darüber hinaus ein Femtosekunden-Lasersystem bekannt, welches ebenfalls den Flap präparieren kann, aber zusätzlich in der Lage ist, den für eine refraktive Korrektur erforderlichen Abtrag stromalen Gewebes durch eine zweifache Schnittführung zur Präparation eines Lentikels zu separieren. Dies kann als Femtosekunden-Lentikel-Extraktion bezeichnet werden. Das Lentikel kann dann nach dem Öffnen des Flaps mit Hilfe einer Pinzette entnommen werden. Dadurch ist nur ein Lasersystem erforderlich, auf einen Excimerlaser kann verzichtet werden.

Problematisch ist, dass durch alle oben beschriebenen Verfahren die visuelle Wahrnehmung aufgrund einer spektralen Aufspaltung einfallenden Weißlichts beeinträchtigt werden kann (Krueger, Thornton, Xu, Bor and van den Berg: "Rainbow Glare as an Optical Side Effect of IntraLasik", Ophthalmology, 2008,115 (7)). Ursache dafür scheinen Defektstellen zu sein, die sich nach der Photodisruption durch den Femtosekunden-Laser an den Fokuspositionen ausbilden und als Transmissionspunktgitter wirken. Auf den cornealen Flaps bleibt die Gitterstruktur zeitweise vorhanden. Durch Verwendung einer Fokussieroptik mit höherer numerischer Apertur kann die Stärke des Effekts vermindert werden, da auf diese Weise kleinere Disruptionsblasen erzeugt werden.

Der Erfindung liegt die Aufgabe zugrunde, ein ophthalmologisches Lasersystem der eingangs genannten Art so zu verbessern, dass die visuelle Wahrnehmung nach der Behandlung weniger oder gar nicht beeinträchtigt ist.

Die Aufgabe wird gelöst durch ein ophthalmologisches Lasersystem, welches die in Anspruch 1 angegebenen Merkmale aufweist.

Vorteilhafte Ausgestaltungen der Erfindung sind in den Unteransprüchen angegeben. Erfindungsgemäß ist vorgesehen, dass Bestrahlungssteuerdaten für Photodisruptionen an mehreren Bestrahlungspunkten im Inneren der Cornea anhand mindestens eines vorgegebenen Soll-Schnittes derart ermittelt werden, dass die Bestrahlungspunkte dreidimensional (insbesondere längs einer optischen Achse oder einer Sehachse) verteilt und nicht-äquidistant angeordnet sind. In einem weiteren Schritt wird das okuläre Gewebe gemäß den ermittelten Bestrahlungssteuerdaten bestrahlt. Mit anderen Worten, die Bestrahlungspunkte werden derart angeordnet, dass aus der Bestrahlung nicht-äquidistante Abstände zwischen benachbarten Photodisruptionsblasen und insbesondere zwischen den aus ihnen resultierenden Defektstellen resultieren. Dies wird vorzugsweise mittels eines ophthalmologischen Lasersystems durch eine Steuereinheit ausgeführt. Die dreidimensionale Verteilung und nicht-äquidistante Anordnung der Bestrahlungspunkte kann entweder bei der erstmaligen Erzeugung der Bestrahlungssteuerdaten oder bei einer nachträglichen Optimierung bereits erzeugter Bestrahlungssteuerdaten durchgeführt werden.

Bestrahlungssteuerdaten im Sinne der Erfindung sind Datensätze von Parametern für ein Lasergerät, wobei jedem Bestrahlungspunkt ein entsprechender Datensatz zugeordnet ist. Auch ein einzelner solcher Datensatz wird nachfolgend der Einfachheit halber mit dem Begriff Bestrahlungssteuerdaten bezeichnet. Jeder Datensatz umfasst die Koordinaten des betreffenden Bestrahlungspunktes, beispielsweise ein Koordinatentripel (x/y/z) im Koordinatensystem des Lasers, und vorzugsweise einen Intensitäts- oder Energiewert für den abzugebenden Laserpuls und optional eine Laserpulsfrequenz.

Dreidimensional verteilt bedeutet im Sinne der Erfindung, dass die Photodisruptionsblasen und die daraus resultierenden Defektstellen nicht ausschließlich in einer Ebene angeordnet sind. Insbesondere sollen die Photodisruptionsblasen und die daraus resultierenden Defektstellen nicht ausschließlich in einer zu einer optischen Achse (des Lasers oder des Auges) oder zu einer Sehachse orthogonalen Ebene angeordnet sein. Nicht-äquidistant bedeutet im Sinne der Erfindung, dass eine Mehrheit der Bestrahlungspunkte und damit der zu erzeugenden Photodisruptionsblasen und der daraus resultierenden Defektstellen wechselseitig nicht-gleiche (also unterschiedliche) Abstände aufweisen. Dies gilt für alle drei Dimensionen der Verteilung.

Indem die Bestrahlungspunkte, an denen Photodisruptionen zu erzeugen sind, erfindungsgemäß gezielt in einer beugungsoptimierten nicht-äquidistanten Anordnung ermittelt werden, können Beugungseffekte wie eine unerwünschte konstruktive Interferenz (im Sinne der Erfindung als Störinterferenz bezeichnet) in durch die Cornea hindurchtretendem Licht minimiert oder vermieden werden. Dadurch kann die visuelle Wahrnehmung nach einer Behandlung mit einem Femtosekunden-Laser verbessert werden.

Der Soll-Schnitt ist für eine Erzeugung eines Flaps und/oder eines Lentikels ausgebildet, so dass die Bestrahlungssteuerdaten (primär) mit dem Ziel einer Erzeugung eines Flaps und/oder eines Lentikels ermittelt werden und erfindungsgemäß sekundär für minimale Störinterferenz optimiert werden. So kann die visuelle Wahrnehmung nach lasergestützten Behandlungen der Cornea verbessert werden.

Vorzugsweise werden die Bestrahlungspunkte regelmäßig angeordnet, da eine regelmäßige Anordnung mit geringem Aufwand und deswegen schnell erzeugt werden kann. Insbesondere wird eine erste Teilmenge der Bestrahlungspunkte in einer ersten Ebene quer zu einer optischen Achse äquidistant angeordnet und eine zweite Teilmenge der Bestrahlungspunkte in einer zweiten Ebene quer zu der optischen Achse äquidistant angeordnet, wobei die zweite Ebene von der ersten Ebene um höchstens einen Durchmesser einer zu erzeugenden Photodisruption entfernt liegt. Durch eine solche regelmäßige Anordnung in z-Richtung versetzter Ebenen mit jeweils äquidistant angeordneten Bestrahlungspunkten kann eine nicht-äquidistante, dreidimensionale Verteilung mit besonders geringem Aufwand und hoher Geschwindigkeit erzeugt werden.

In einer besonderen Ausführungsform wird eine dritte Teilmenge der Bestrahlungspunkte in einer dritten Ebene quer zu der optischen Achse äquidistant angeordnet, wobei sich der Abstand zu der ihre näherliegenden der beiden anderen Ebenen von dem Abstand zwischen den beiden anderen Ebenen unterscheidet. Vorteilhafterweise liegt dabei die dritte Ebene von der ihr näherliegenden der beiden anderen Ebenen um höchstens einen Durchmesser einer zu erzeugenden Photodisruption entfernt. Dies verringert die Störinterferenz effizient.

Alternativ können die Bestrahlungspunkte auch statistisch verteilt angeordnet werden. Eine solche statistische Verteilung der Bestrahlungspunkte kann beispielsweise von einer in xyz regulären Struktur mit äquidistanten Abständen zumindest in einer Dimension derart abgeleitet werden, dass z.B. eine Gaußverteilung für jeden Bestrahlungspunkt der zugrunde gelegten äquidistanten Gitterstruktur zugelassen wird, wobei die Halbwertsbreite der Gaußverteilung z.B. kleiner als der halbe Abstand zweier Bestrahlungspunkte in der zugrunde gelegten Gitterstruktur ist.

Damit wird sichergestellt, dass eine Schnittebene entsteht obwohl die Regularität der Anordnung der Bestrahlungspunkte aufgegeben wurde.

Zweckmäßigerweise werden zum Ermitteln der Bestrahlungssteuerdaten folgende Schritte ausgeführt:
a) Ermitteln von vorläufigen Bestrahlungssteuerdaten gemäß dem mindestens einen Soll-Schnitt,
b) Prognostizieren eines aus den vorläufigen Bestrahlungssteuerdaten bei durchfallendem Licht resultierenden Interferenzmusters,
c) Ermitteln einer Intensität von Beugungslicht in der ersten oder einer höheren Ordnung des prognostizierten Interferenzmusters,
d) Vergleichen der ermittelten Intensität mit einem vorgegebenen Intensitätsschwellwert und
e) Wiederholen der Schritte a) bis d), wenn die ermittelte Intensität größer als der Intensitätsschwellwert ist, wobei in Schritt a) die vorläufigen Bestrahlungssteuerdaten modifiziert oder andere vorläufige Bestrahlungssteuerdaten ermittelt werden. Diese iterativen Schritte ermöglichen es, beugungsoptimierte Bestrahlungssteuerdaten mit geringem Aufwand und in kurzer Zeit zu ermitteln. Zweckmäßigerweise wird in Schritt c) ein lokales Maximum (engl. "peak") innerhalb der betreffenden Beugungsordnung ermittelt und die Intensität an dieser Stelle für den Vergleich in Schritt d) verwendet.

Vorteilhafterweise ist eine Anordnung zur konfokalen Detektion von Strahlung aus dem Untersuchungsbereich vorgesehen. Der Detektionsstrahlengang kann beispielsweise Teil eines konfokalen Laser-Scanning-Mikroskops (LSM) sein, das zusätzlich zu dem Behandlungslaser vorgesehen ist. Die konfokale Detektion ermöglicht die Vermessung des okulären Gewebes, insbesondere seine inneren Eigenschaften wie Lage, Form, Brechungsindexverteilung und Zusammensetzung, mit hoher Genauigkeit.

Im Falle der Cornea können durch konfokale Detektion beispielsweise Form und/oder Lage ihrer posterioren Grenzfläche ermittelt werden. Die posteriore Grenzfläche ist der Übergang vom Endothel, dem posterioren Abschluss der Cornea, zum Kammerwasser des Auges. An dieser Mediengrenze treten ein Brechzahlsprung und eine erhöhte Reflexion auf, die beispielsweise mit einem konfokalen Detektor mit hoher Genauigkeit gemessen werden können. Alternativ können Messgeräte wie eine Scheimpflug-Kamera oder ein OCT-System verwendet werden, die jedoch mehr Aufwand erfordern. Durch die Vermessung der posterioren Grenzfläche kann diese vorteilhafterweise als Bezugsfläche für keratoplastische Schnitte genutzt werden.

In einer weiteren vorteilhaften Ausgestaltung ist ein Strahlteiler vorgesehen, der Strahlung, die den Strahlteiler aus Richtung des Untersuchungsbereichs erreicht, durch eine konfokale Aperturblende als Detektionslicht auf einen Detektor leitet. Auf diese Weise können der Beleuchtungsstrahlengang und der Detektionsstrahlengang mit hoher Genauigkeit aufeinander ausgerichtet sein. Dieselben optischen Elemente (Fokussieroptik usw.) können dadurch doppelt genutzt werden. Bevorzugt sind dabei Ausführungsformen, in denen die Strahlung des Lasers neben der Beleuchtungs-Laserleistung auf eine chirurgische Therapie-Laserleistung einstellbar ist. Dadurch ist derselbe Laser sowohl für die Beleuchtung bei der Ermittlung von Form und/oder Lage des zu behandelnden okulären Gewebes als auch bei der anschließenden Behandlung mit hoher Positioniergenauigkeit verwendbar. Die Verwendung desselben Lasers zur Vermessung und Behandlung ermöglicht dabei eine hohe Genauigkeit der Positionierung des Behandlungsfokus, da die Vermessung im quasiabsoluten Bezugssystem des Behandlungslasers erfolgen kann.

Der Strahlteiler kann vorteilhaft als Polarisationsstrahlteiler ausgebildet sein, der das Detektionslicht selektiv so auf den Detektor auskoppelt, dass es eine von dem emittierten Beleuchtungslicht verschiedene Polarisationsrichtung aufweist. Ein großer Teil des vom Untersuchungsbereich auf den Strahlteiler treffenden Lichts stammt aus Reflexionen an den optischen Komponenten des Strahlengangs, beispielsweise den Oberflächen der Fokussieroptik, und weist daher dieselbe Polarisationsrichtung auf wie das Beleuchtungslicht. Indem der Strahlteiler nur Licht einer davon verschiedenen Polarisationsrichtung als Detektionslicht zum Detektor leitet, wird solches Störlicht zurückgehalten. In der Cornea oder in der Augenlinse rückgestreutes Licht hingegen weist eine veränderte Polarisationsrichtung auf. Die Detektion des in der Cornea rückgestreuten Lichts ist dadurch mit höherer Genauigkeit möglich.

Die Aufgabe der Erfindung wird auch gelöst durch ein ophthalmologisches Lasersystem, welches die in Anspruch 8 angegebenen Merkmale aufweist.

In dieser weiteren Ausprägung der Erfindung werden Bestrahlungssteuerdaten für Photodisruptionen an mehreren Bestrahlungspunkten im Inneren des okulären Gewebes anhand mindestens eines vorgegebenen Soll-Schnittes derart ermittelt, dass die Bestrahlungspunkte regelmäßig angeordnet sind und durch konstruktive Interferenz in durch das okuläre Gewebe hindurchtretendem Licht eine Kompensation eines refraktiven Defizits resultiert. In einem weiteren Schritt wird das okuläre Gewebe gemäß den ermittelten Bestrahlungssteuerdaten bestrahlt. Dies wird vorzugsweise mittels eines ophthalmologischen Lasersystems durch eine Steuereinheit ausgeführt. Beispielsweise werden Zonen mit Bestrahlungspunkten entsprechend einer Fresnelschen Stufenlinse und/oder entsprechend einer Fresnelschen Zonenlinse angeordnet. Das erfolgt vorzugsweise zusätzlich zu einem primären Bestrahlungsziel wie einem Flap- oder Lentikelschnitt oder einer Auflockerung einer presbyopen Augenlinse.

Indem die Bestrahlungspunkte, an denen Photodisruptionen zu erzeugen sind, gezielt in einer interferenzerzeugenden regelmäßigen Anordnung ermittelt werden, können an sich unerwünschte Beugungseffekte zur Verbesserung einer Sehschwäche genutzt werden. Dadurch kann insbesondere eine anderweitige Störinterferenz unterdrückt werden. Realisiert werden kann dies beispielsweise im Falle einer presbyopen Augenlinse, indem die Photodisruptionen zur Auflockerung der verhärteten Linse gerade so erzeugt werden, dass gezielt Beugungseffekte resultieren, die ein refraktives Defizit des Auges kompensieren. Im Stand der Technik ist es lediglich bekannt, künstliche Intraokularlinsen auf der Oberfläche mit refraktiven und/oder diffraktiven Strukturen zu versehen (Dissertation Roman Grolmus, Gießen 2006, VVB Laufersweiler Verlag). Gemäß der Erfindung wird zwar eine entsprechende optische Wirkung erzielt. Erfindungsgemäß wird dieser Effekt jedoch in vivo durch Photodisruption im Inneren der Linse erzielt, wobei lokale Brechzahlunterschiede zwischen den Photodisruptionsblasen (Kavitäten) ausgenutzt werden. Auf die Implantation einer künstlichen Intraokularlinse kann verzichtet werden.

In bevorzugten Ausgestaltungen ist im Falle einer presbyopen Augenlinse als zu behandelndem okulärem Gewebe der Soll-Schnitt für eine Auflockerung des Linsengewebes ausgebildet, so dass die Bestrahlungssteuerdaten (primär) mit dem Ziel einer Auflockerung des Linsengewebes ermittelt und erfindungsgemäß dabei oder anschließend sekundär für eine minimale Störinterferenz optimiert werden. Die Bestrahlungspunkte werden im Fall der Augenlinse so ermittelt, dass der lasergestützte Energieeintrag vollständig im Inneren der Linse erfolgt. Im Falle einer Cornea ist der Soll-Schnitt für eine Erzeugung eines Flaps und/oder eines Lentikels ausgebildet, so dass die Bestrahlungssteuerdaten (primär) mit dem Ziel einer Erzeugung eines Flaps und/oder eines Lentikels ermittelt werden und erfindungsgemäß sekundär für minimale Störinterferenz optimiert werden. So kann die visuelle Wahrnehmung nach lasergestützten Behandlungen der Augenlinse beziehungsweise der Cornea verbessert werden. Beide Fälle können kombiniert werden, indem in derselben Operation sowohl die Cornea als auch die Linse behandelt wird.

In bevorzugten Ausgestaltungen werden die Bestrahlungssteuerdaten so ermittelt, dass mindestens drei Fokusebenen in unterschiedlichen Entfernungen resultieren. Dies gelingt durch eine sequentielle Anordnung von interferenzerzeugenden Bestrahlungspunktmustern in Art einer Reihenschaltung.

In einem weiteren Beispiel werden Bestrahlungssteuerdaten für Photodisruptionen an mehreren Bestrahlungspunkten im Inneren des okulären Gewebes anhand mindestens eines vorgegebenen Soll-Schnittes derart, dass für zumindest einige der zu erzeugenden Photodisruptionen unterschiedliche räumliche Ausdehnungen resultieren. Später erfolgt die Bestrahlung des okulären Gewebes gemäß den ermittelten Bestrahlungssteuerdaten. Erfindungsgemäß wurde erkannt, dass Interferenzerscheinungen durch variierende räumliche Ausdehnungen der Photodisruptionen vermieden werden können, da dadurch die Defektstellen als Streuzentren unterschiedliche Größen aufweisen. Diese Art der Beugungsoptimierung ist auch bei auf herkömmliche Weise äquidistant angeordneten Bestrahlungspunkten/Photodisruptionen/Defektstellen anwendbar. Die unterschiedlichen räumlichen Ausdehnungen können mit geringem Aufwand insbesondere durch eine Variation einer Pulsenergie zwischen den betreffenden Bestrahlungspunkten und/oder durch Anordnung einer variierenden Anzahl von Bestrahlungspunkten zur Überlappung der resultierenden Photodisruptionen erzielt werden.

Die nachfolgend beschriebenen weitergehenden Ausgestaltungen sind sowohl in Ausprägungen mit nicht-äquidistanten Bestrahlungspunkten zur Interferenzminimierung als auch in Ausprägungen mit regelmäßigen, gezielt refraktiv und/oder diffraktiv wirkenden, also interferenzerzeugenden Bestrahlungspunkten anwendbar.

Vorzugsweise werden die Bestrahlungspunkte als auf einer durchgehenden Raumkurve liegend ermittelt. Insbesondere können sich zwei aufeinanderfolgende Bestrahlungspunkte in allen drei Raumkoordinaten unterscheiden, wodurch die Strahlenbelastung reduziert werden kann. Zudem ermöglicht dies, die Behandlung in kurzer Zeit abzuschließen. Technisch besonders vorteilhaft ist eine Ansteuerung der x-y-Scanner in Form von harmonischen Schwingungen, insbesondere Sinus-Funktionen. Dieser Bewegung kann zusätzlich eine Bewegung des Bestrahlungsfokus in z-Richtung überlagert werden.

Vorteilhafterweise wird vor dem Ermitteln von Bestrahlungssteuerdaten mindestens eine Eigenschaft des okulären Gewebes ermittelt und bei der Ermittlung der Bestrahlungssteuerdaten und/oder bei einer Korrektur der Bestrahlungssteuerdaten berücksichtigt. Dies ermöglicht eine hohe Genauigkeit der Bestrahlung. Beispielsweise kann die Gradientenindexstruktur der Augenlinse als Eigenschaft ermittelt und berücksichtigt werden. Dies gilt insbesondere für die äquidistante Anordnung von Bestrahlungspunkten zum Zwecke der Kompensation einer Sehschwäche mittels Fresnelzonen oder -stufen.

Auch für eine solche, der Bestrahlungssteuerdatenerstellung oder -korrektur vorausgehende optische Ermittlung von Eigenschaften des okulären Gewebes mittels konfokaler Detektion können die Abtastpunkte auf einer durchgehenden Raumkurve liegen. Bei einer Ansteuerung der x-y-Scanner in der Form, dass der eine Scanner mit genau der doppelten Frequenz des anderen angesteuert wird, ergibt sich beispielweise eine Lissajous-Figur, die einer Acht ähnelt. Dieser Bewegung kann zusätzlich eine Bewegung des Bestrahlungsfokus in z-Richtung überlagert werden. Dadurch können sich zwei aufeinanderfolgende Abtastpunkte in allen drei Raumkoordinaten unterscheiden, wodurch die Strahlenbelastung reduziert werden kann. Darüber hinaus kann die Pulsfrequenz des Laserlichts in Abhängigkeit der Bewegungsgeschwindigkeit des Fokuspunkts des Laserstrahls relativ zum okulären Gewebe eingestellt werden. Dadurch kann bei der Ermittlung von Gewebeeigenschaften und auch bei der eigentlichen Behandlung die Strahlenbelastung des okulären Gewebes und des Auges insgesamt verringert werden.

Vorzugsweise wird bei der Ermittlung der Bestrahlungssteuerdaten und/oder bei einer Korrektur der Bestrahlungssteuerdaten eine bezüglich des okulären Gewebes positionsabhängige Effizienz der photodisruptiven Bestrahlung berücksichtigt. Bei eigenen Untersuchungen wurde festgestellt, dass es insbesondere in Abhängigkeit von der z-Position zu Unterschieden in der Wechselwirkung der Laserstrahlung mit dem Linsengewebe kommt. Diese Abhängigkeit kann durch Anpassung der Abstände der Bestrahlungspunkte (Spotabstand) und der Strahlenergie kompensiert werden. Dadurch kann die Genauigkeit der Behandlung verbessert werden. Insbesondere kann die positionsabhängige Effizienz im Rahmen eines Korrekturschritts in zuvor erstellten Bestrahlungssteuerdaten berücksichtigt werden.

Eine nicht-äquidistante Anordnung der Bestrahlungspunkte wird vorteilhafterweise mittels eines vor oder nach einer Laserverstärkungsanordnung angeordneten Pulswählers (engl. "pulse picker") durch zeitlich nicht-äquidistante Steuersignale erzeugt. Ein solcher Pulswähler ist in der Regel ohnehin vor jeder Verstärkerstufe zur Heruntertaktung der jeweiligen Eingangsstrahlung vorgesehen. Die Taktfrequenz des Pulswählers kann durch eine geeignete Ansteuerung variiert werden, um die Pulsfrequenz am Laserausgang in gewünschter Weise zu einzustellen. Dies ermöglicht die Anpassung der Abstände der Bestrahlungspunkte mit geringem Aufwand. Realisiert werden kann dies durch eine modifizierte Ansteuerung des Pulswählers. Üblicherweise erfolgt die Ansteuerung mit einem festen Tastverhältnis, das heißt, es werden zum Beispiel 99 Pulse in Folge geblockt und nur jeder hundertste Puls wird transmittiert. Um den gewünschten Effekt zu erzielen, kann dieses Verhältnis variiert werden, beispielsweise zwischen 80:1 und 120:1. Die Variation kann dabei durch ein Zufallsprinzip bestimmt werden oder regelmäßig erfolgen. Die Steuersignale für den Pulswähler können beispielsweise aus den Koordinaten jeweils zweier aufeinanderfolgender Bestrahlungspunkte ermittelt werden. Mittels eines Pulswählers kann vorteilhafterweise zusätzlich auch die Pulsenergie moduliert werden, indem der zeitliche Abstand zwischen den Pulsen eingestellt wird. Beispielsweise entsteht bei einem größeren zeitlichen Abstand eine höhere Pulsenergie.

Die Erfindung ist in den Ansprüchen definiert. Andere Ausführungsbeispiele sind lediglich für illustrative Zwecke beschrieben.

Nachfolgend wird die Erfindung anhand von Ausführungsbeispielen näher erläutert.

In den Zeichnungen zeigen:
Fig. 1 ein Schema eines ophthalmologischen Lasersystems zur Analyse und Bearbeitung der Augenlinse und/oder der Cornea,
Fig. 2 eine Raumkurve für die Abtastung der Augenlinse,
Fig. 3 ein Ablaufdiagramm eines Betriebsverfahrens für ein ophthalmologisches System,
Fig. 4 eine schematische Darstellung der Wirkungsweise einer beugungsoptimierten Anordnung von Kavitäten,
Fig. 5 eine schematische Darstellung von unregelmäßig angeordneten Defektstellen,
Fig. 6 eine schematische Darstellung einer ersten optimierten Schnittanordnung,
Fig. 7 eine schematische Darstellung einer zweiten optimierten Schnittanordnung,
Fig. 8 eine schematische Darstellung einer ersten regelmäßigen, nicht-äquidistanten Anordnung von Kavitäten,
Fig. 9 eine schematische Darstellung einer zweiten regelmäßigen, nicht-äquidistanten Anordnung von Kavitäten,
Fig. 10 eine schematische Darstellung einer dritten regelmäßigen, nicht-äquidistanten Anordnung von Kavitäten,
Fig. 11 ein Ablaufdiagramm eines weiteren Betriebsverfahrens für ein ophthalmologisches System und
Fig. 12 den inneren Brechzahlgradienten von Augenlinsen.

In allen Zeichnungen tragen übereinstimmende Teile gleiche Bezugszeichen.

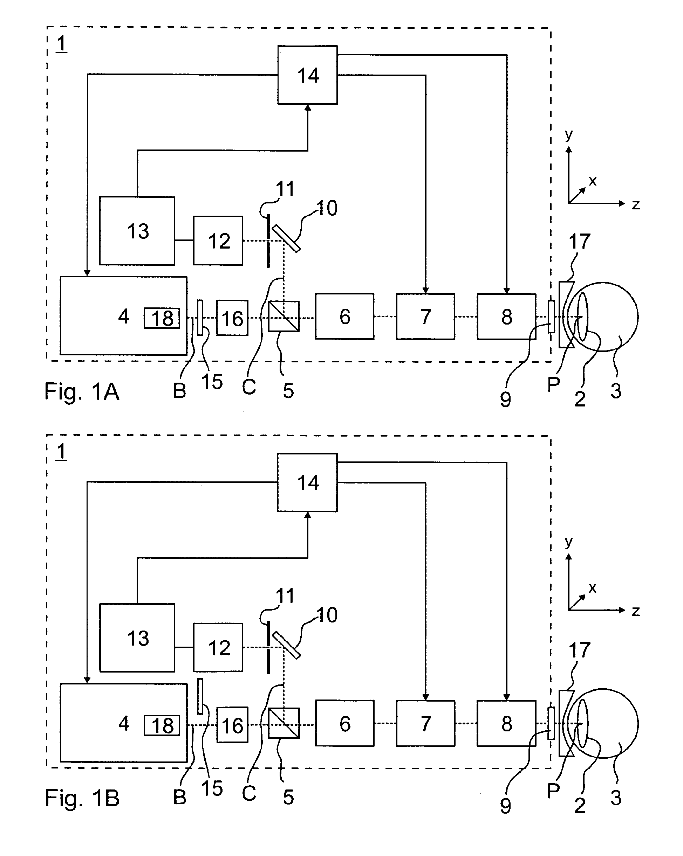

**Fig. 1** zeigt ein beispielhaftes ophthalmologisches Lasersystem 1 zur kombinierten Analyse und Therapie einer Presbyopie einer Augenlinse 2 eines Auges 3. Mit diesem System kann auch die Cornea (nicht abgebildet) des Auges 3 behandelt werden. Das Lasersystem 1 weist im Beleuchtungsstrahlengang B einen Laser 4, einen Polarisationsstrahlteiler 5, eine Scanoptik 6, eine Scannereinheit 7, eine Fokussieroptik 8 und ein optisches Phasenverzögerungssystem 9 auf, sowie einen Umlenkspiegel 10, eine konfokale Aperturblende 11 und einen Detektor 12, die einen ausgekoppelten Detektionsstrahlengang C bilden, und einen Verstärker 13 und eine Steuereinheit 14. Im Beleuchtungsstrahlengang ist zudem ein Modulator 16, beispielsweise ein akustooptischer Modulator (AOM, AOTF), vorgesehen. Zwischen dem Lasersystem 1 und dem Auge 3 ist ein Kontaktglas 17 mit einer Fixiervorrichtung für das Auge 3 angeordnet, hinter dem der Untersuchungsbereich liegt. Andere Ausführungsformen zur Realisierung der erfindungsgemäßen Lösung sind möglich (nicht abgebildet). Beispielsweise kann ein eigenständiger Detektionsstrahlengang verwendet werden, insbesondere in Form eines konfokalen Mikroskops. Die Ermittlung von Eigenschaften des zu behandelnden Gewebes (Augenlinse, Cornea) durch optische Detektion muss dabei nicht in zeitlichem Zusammenhang mit der Behandlung erfolgen. Sie kann auch präoperativ erfolgen.

Der Laser 4 ist beispielsweise als gepulster TiSa-Infrarot-Laser mit einer Pulslänge zwischen 100 fs und 1000 fs ausgebildet. In den Beleuchtungsstrahlengang B ist ein Abschwächer 15 einklappbar. Er dient zur Umschaltung zwischen einer Beleuchtungs-Laserleistung und einer Therapie-Laserleistung. Die Beleuchtungs-Laserleistung wird mit in den Beleuchtungsstrahlengang B eingeklapptem Abschwächer 15 erzielt (Teilfigur 1A), die Therapie-Laserleistung ohne Abschwächer 15 (Teilfigur 1 B). Die optischen Komponenten, insbesondere die Optiken 6 und 8, sind auf das Ziel einer bestmöglichen Fokusminiaturisierung optimiert, korrigiert und untereinander abgestimmt. Beispielsweise sind ihre optischen Aberrationen hochgradig minimiert, so dass für eine Photodisruption nur ein geringer Energieeintrag erforderlich ist. Die optischen Komponenten sind derart gestaltet, dass die Eigendispersion der intraokularen Medien bezüglich der Pulslängenänderung und auch die eigenfokussierende Wirkung der Gradientenlinsenstruktur der Augenlinse vorkompensiert werden. Dadurch kann die Größe des Fokusvolumens über die gesamte Fläche der Augenlinse hinweg und über ihre gesamte Tiefe mit einem Fehler von maximal 10% konstant gehalten werden.

Die Scannereinheit 7 umfasst beispielsweise eine Anzahl von galvanometrischen Spiegeln zur Ablenkung der Laserstrahlung in x- und y-Richtung über die Augenlinse 2. Die Fokussierung der Laserstrahlung in z-Richtung entlang der optischen Achse gelingt beispielsweise durch eine bewegliche Linse oder Linsengruppe innerhalb der Scanoptik 6 oder der Fokussieroptik 8 oder durch eine bewegliche Tubuslinse (nicht abgebildet). Das optische Phasenverzögerungssystem 9 ist beispielsweise als λ/4-Platte ausgebildet, die einen Abschluss des Lasersystems bildet. Der Detektor 12 ist beispielsweise als Photomultiplier oder als APD ausgebildet, da die aufzunehmenden Lichtintensitäten niedrig sind. Der Verstärker 13 ist als Lock-In-Verstärker ausgebildet und sowohl mit dem Detektor 12 als auch mit dem Laser 4 verbunden. Der Laser 4 ist intern mit einem Pulswähler 18 zur Erzeugung zeitlich variabler Abstände zwischen aufeinanderfolgenden Laserpulsen ausgestattet. Die Steuereinheit 14 kann die zeitlichen Abstände über entsprechende Steuersignale einstellen.

Die gepulste IR-Laserstrahlung tritt aus dem Laser 4 aus und durchläuft zunächst unverändert den Polarisationsstrahlteiler 5. Sie wird anschließend über die Scanoptik 6, die Scannereinheit 7 und die Fokussieroptik 8 als Beleuchtungslicht auf einen Abtastpunkt P in der Augenlinse 2 fokussiert. Dieser Abtastpunkt P kann mittels der Scannereinheit 7 und einer beweglichen Linse oder Linsengruppe innerhalb der Scanoptik 6 oder der Fokussieroptik 8 in x-, y- und z-Richtung in der Augenlinse 2 verschoben werden. Das optische Phasenverzögerungssystem 9 bewirkt dabei eine definierte Veränderung der Polarisationsrichtung des hindurchfallenden Beleuchtungslichts.

An den Grenzflächen der Augenlinse 2 und an inhomogenen Schichten der Augenlinse (nicht abgebildet) kommt es zur Streuung/Reflexion der IR-Strahlung, wobei die Strahlung im Auge 3 teilweise depolarisiert wird. Dies gilt bei einer Behandlung Cornea entsprechend auch für die Grenzflächen und das Innere der Cornea. Rückgestreutes/reflektiertes Licht fällt in den Beleuchtungsstrahlengang B und läuft dort den Weg zurück bis zum Polarisationsstrahlteiler 5. Die Strahlungsanteile mit unverändertem Polarisationszustand fallen durch den Polarisationsstrahlteiler 5 hindurch auf den Laser 4. Dies betrifft insbesondere Reflexe, die von der Scanoptik 6 oder der Fokussieroptik 8 stammen. Solche Strahlungsanteile, die nach Durchlaufen des Phasenverzögerungssystems 9 beziehungsweise durch Depolarisation im Auge 3 in der Augenlinse 2 einen veränderten Polarisationszustand aufweisen, werden von dem Polarisationsstrahlteiler 5 als Detektionslicht in den Detektionsstrahlengang C zum Detektor 12 abgelenkt. Das Detektionslicht fällt über einen Umlenkspiegel 10 durch die konfokale Lochblende 11 auf den Detektor 12. Der Umlenkspiegel 10 kann in einer Ausführungsform entfallen oder durch andere Strahlführungseinheiten ersetzt werden. Die Konfokalblende 11 wirkt als Diskriminator in z-Richtung, so dass ortsaufgelöst nur rückgestreutes Licht aus einem geringen Fokusvolumen detektiert wird. Die Steuereinheit 14 kann durch Ablenken des Beleuchtungslichts in x- und y-Richtung mittels der Scannereinheit 7 und Veränderung der Fokussierung in z-Richtung mittels der Fokussieroptik 8 beliebige Abtastpunkte P innerhalb und außerhalb der Augenlinse 2 mit Beleuchtungslicht bestrahlen und über die Intensität des zugehörigen Detektionslichtes die Stärke der Rückstreuung an diese Punkten ermitteln.

In der abgebildeten Ausführungsform bewirkt das optische Phasenverzögerungssystem 9 zwischen dem Auge 3 und der Fokussieroptik 8 eine definierte Drehung der Polarisationsrichtung der hindurchfallenden Beleuchtungslichts, während zuvor an den optischen Komponenten reflektiertes Störlicht die ursprüngliche Polarisationsrichtung beibehält. Dadurch wird die relative Intensität des Detektionslichtes erhöht, da der Polarisationsstrahlteiler 5 jegliches Licht mit abweichender Polarisationsrichtung als Detektionslicht separiert. In alternativen Ausführungsformen kann auf das optische

Phasenverzögerungssystem 9 verzichtet werden. Alternativ oder zusätzlich können weitere Polarisatoren (nicht abgebildet) in dem Beleuchtungs- und/oder Detektionsstrahlengang angeordnet werden, um die Signalqualität zu verbessern. Das Phasenverzögerungssystem kann in einer Ausführungsform als Depolarisator realisiert werden, so dass über den Strahlquerschnitt die Größe der Phasenverzögerung variiert.

Da die am Detektor 12 registrierten Signale eine sehr geringe Intensität aufweisen, ist der elektronische Verstärker für ein optimiertes Signal-Rausch-Verhältnis angepasst. Eine besonders vorteilhafte Ausführungsform ist der Lock-In-Verstärker, der zeitlich mit der Pulsgenerierung beziehungsweise mit der Folgefrequenz des Lasers 2 synchronisiert ist. Andere Ausführungsformen verwenden beispielsweise sogenannte "Boxcar"-Techniken oder Abtasttechniken (engl. "sampling") mit Aufsummierung oder Mittelung. Vorteilhafterweise weist das gesamte Verstärkersystem des Detektorsignals eine nichtlineare Kennlinie auf.

Zweckmäßigerweise wird das Patientenauge 3 vor einer Detektion und/oder Therapie fixiert, beispielsweise mittels Unterdruck an die Kontaktglasvorrichtung 17 angesaugt. Zusätzlich kann der Kopf des Patienten fixiert sein (nicht abgebildet). Der Blick des Patienten kann durch ein geeignetes Fixierziel (nicht abgebildet) möglichst konstant gehalten werden. Dabei ist eine einstellbare Kompensation des Winkels zwischen Geometrie- und Sehachse des Auges möglich.

Zur Ermittlung von Eigenschaften des zu behandelnden okulären Gewebes (Augenlinse, Cornea) kann das Beleuchtungslicht bei Beleuchtungs-Laserleistung (Abschwächer 15 befindet sich im Beleuchtungsstrahlengang B) mit einer variablen Pulsfrequenz entlang einer einstellbaren kontinuierlichen, dreidimensionalen Abtastkurve oder -struktur über die Augenlinse 2 (oder die Cornea) geführt werden, wobei Detektionslicht aufgenommen wird. Die Pulsfrequenz wird dabei beispielsweise in Abhängigkeit der Geschwindigkeit der Abtastbewegung so eingestellt, dass bei langsamer Abtastbewegung eine niedrigere Pulsfrequenz resultiert als bei schneller Abtastbewegung. Einzelnen Punkten der Abtastkurve wird das rückgestreute Detektionslicht abschnitts- oder punktweise zugeordnet. Durch die Stetigkeit der Abtastkurve unterscheiden sich aufeinanderfolgende Abtastpunkte in allen Raumkoordinaten. Von den detektierten Signalwerten werden vorteilhafterweise jeweilige Dunkelfeldwerte abgezogen werden, die in einem separaten Kalibrierdurchgang ermittelt werden können.

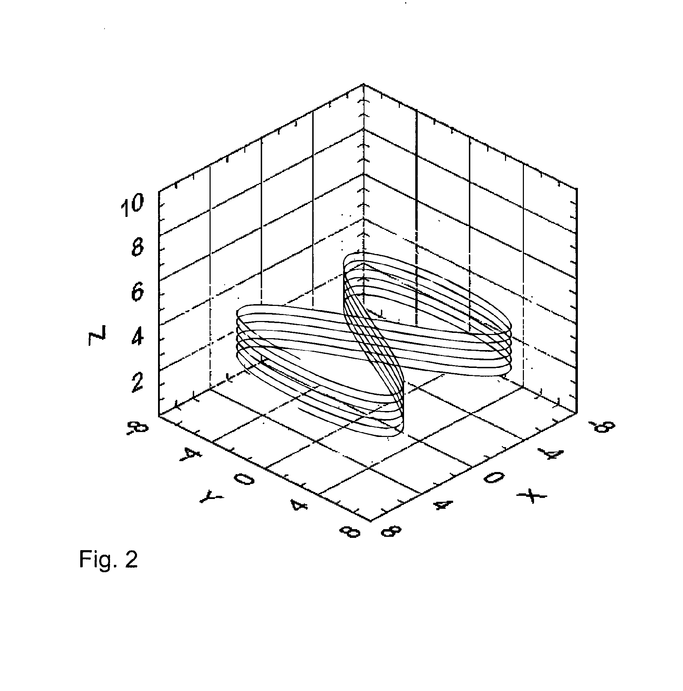

In **Fig. 2** ist eine beispielhafte Abtastkurve in Form räumlich versetzter Achten gezeigt, die als Lissajous-Figur mittels der Scannereinheit 6 realisiert werden kann. Sie hat den Vorteil, dass in kurzer Zeit repräsentative Daten für die Rekonstruktion eines Linsenmodells (oder eines Modells der Cornea) mit hoher Genauigkeit ermittelt werden können. Andere beispielhafte Formen der Abtastung beziehungsweise Abrasterung können sein (nicht abgebildet): zwei gekreuzte Rechtecke im Raum; zwei Zylinderoberflächen; ein zylindrischer Körper mit Querschnitt in Form einer acht oder vier; mehrere Abtastungen längs eindimensionaler Linien. Möglich ist auch das Abrastern des Volumens eines Zylinders oder eines Würfels. Die Volumina beziehungsweise Oberflächen können kontinuierlich oder auch lediglich teilweise, also mit Zwischenräumen zwischen den einzelnen Abtastpunkten, abgerastert werden. So können zwischen einzelnen Zeilen größere Abstände auftreten. Die Abrasterungsstruktur erstreckt sich vorteilhafterweise von den Grenzen her über einen Bereich von mindestens 2,5 mm bis 5 mm axial hinter dem Kontaktglas und von mindestens 0 mm bis 4 mm im Durchmesser lateral in Bezug auf die optische Achse der Bearbeitungsoptik.

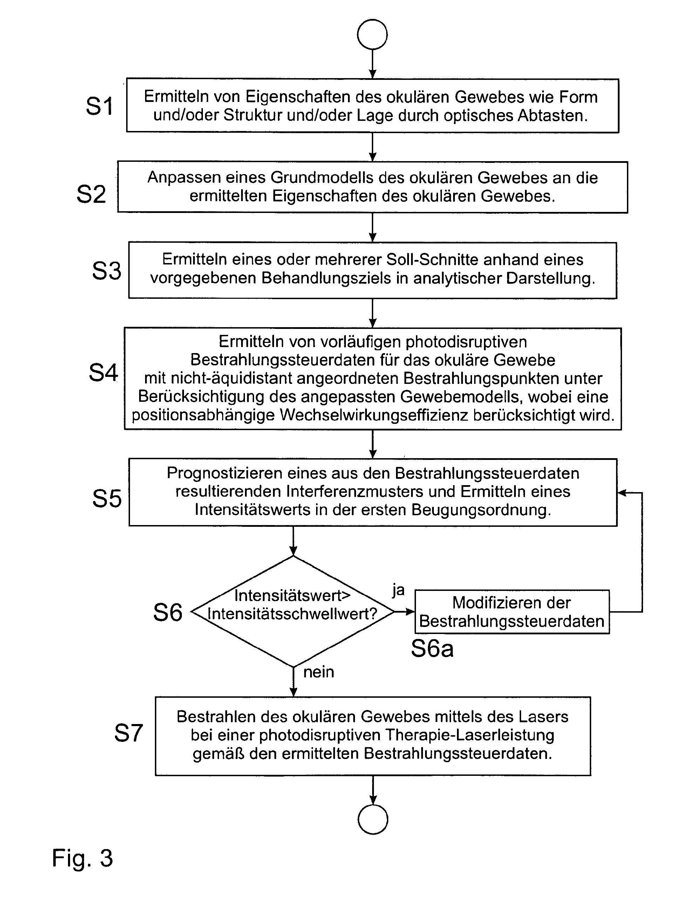

Zur Behandlung einer Presbyopie führt die Steuereinheit 14 beispielsweise das in **Fig. 3** dargestellte Betriebsverfahren durch. Dabei wird derselbe Laser 4 verwendet wie für die Beleuchtung während der Detektionsphase, jedoch zur Photodisruption ohne Zwischenschaltung des Abschwächers 15. Insbesondere kann sich die Therapiephase unmittelbar an die Detektionsphase anschließen. Es ist auch möglich, die Detektionsphase zwischen den nachfolgend genannten Schritten durchzuführen, insbesondere auch wiederholt.

Aus den den Abtastpunkten zugeordneten Intensitäten können beispielsweise die Form, die Struktur und die Lage der Augenlinse 2 (oder der Cornea) rekonstruiert werden (Schritt S1). *Dazu können insbesondere ihre Grenzflächen identifiziert werden, beispielsweise die vordere oder hintere Grenzfläche und*/*oder innere Flächen wie der Übergang zwischen Kortex und Nukleus. Diese Informationen werden verwendet, um ein Grundmodell der Augenlinse (oder Cornea) an den tatsächlichen, individuellen Zustand der Augenlinse 2 anzupassen (Schritt S2). Grundmodelle können beispielsweise Kugeloberflächen, Ellipsoide oder Kegelschnitte sein, die an die rekonstruierten Eigenschaften wie Form, Struktur und*/*oder Lage angepasst werden, beispielsweise durch Verschiebung, Verkippung, Beschneidung der Grenzen, Vergrößerung oder Dehnung , um eine Zentrierung in Bezug auf die reale Lage der Linse im Raum sowie eine Einhaltung von Sicherheitszonen zu ermöglichen.* Das angepasste Modell kann beispielsweise zusätzlich die Augenlinse 2 als Gradientenlinse, also mit einem inneren Verlauf der Brechzahl des Linsenmediums, darstellen. Das Modell kann insbesondere eine Verkippung der Augenlinse 2 zur optischen Achse des Systems 1 wiedergeben. Im Falle der Behandlung der Cornea können als Eigenschaften des okulären Gewebes beispielsweise ihre vordere und/oder ihre hintere Grenzfläche zur Dickenermittlung und als Bezugsfläche(n) identifiziert werden.

Unter Verwendung eines patientenindividuell angepassten Modells wird gemäß einem eigentlichen Behandlungsziel, beispielsweise einer Auflockerung der Augenlinse (oder einem Flap- oder Lentikelschnitt in der Cornea), eine Menge von Soll-Schnitten ermittelt (Schritt S3). Dies kann beispielsweise durch Entgegennahme einer manuellen Eingabe eines Bedieners, insbesondere eines Arztes, oder durch ein Computerprogramm nach Vorgaben des Bedieners erfolgen. Die Soll-Schnitte können beispielsweise in analytischer Form, insbesondere als parametrisierte Funktionen, dargestellt sein und werden in dieser Form weiterverarbeitet.

Zunächst werden anhand der vorgegebenen Soll-Schnitte auf bekannte Weise vorläufige diskrete photodisruptive Bestrahlungssteuerdaten zur Auflockerung der presbyopen Augenlinse 2 mit nicht-äquidistant angeordneten, beugungsoptimiert verteilten Bestrahlungspunkten ermittelt (Schritt S4). Die Bestrahlungssteuerdaten umfassen beispielsweise Ansteuersignale für die x- und y-Achsen der Scannereinheit beziehungsweise für die interne z-Fokussierung und für die Laserstrahlquelle und den Leistungsmodulator 16 sowie für den Pulswähler 18. Beispielsweise wird zur Ermittlung der nicht-äquidistanten Bestrahlungssteuerdaten für jeden Soll-Schnitt ein Grundmuster von Bestrahlungspunkten nach Art und Geometrie des betreffenden Soll-Schnittes aus einer vorgegebenen Bibliothek von beugungsoptimierten Grundmustern ausgewählt. Jedes Grundmuster enthält beispielsweise einen Ausgangswert für einen mittleren Abstand und eine mittlere Streuung der Abstände zwischen den Bestrahlungspunkten, anhand dessen die Auswahl erfolgen kann. Zu jedem Grundmuster enthält die Bibliothek darüber hinaus Vorgaben für mögliche Modifikationsstufen zur Verringerung der Störinterferenz. Neben Grundmustern mit parametrisierten Anordnungsregeln können auch solche mit zufälliger Anordnung der Bestrahlungspunkte verwendet werden.

Anschließend werden die Bestrahlungssteuerdaten hinsichtlich der Beugungswirkung der resultierenden Defektstellen optimiert. Zu diesem Zweck wird durch eine Simulation prognostiziert, wie stark ein Interferenzmuster bei durch das okuläre Gewebe fallendem Licht auftreten würde, wenn die vorläufigen Bestrahlungssteuerdaten in das Gewebe übertragen würden (Schritt S5). Die Stärke des Interferenzmusters wird anhand eines Intensitätsmaximums in einer beispielhaften ersten Beugungsordnung beurteilt und mit einem vorgegebenen maximalen Intensitätsschwellwert verglichen (Schritt S6). Bei zu großer Interferenzintensität wird die Ermittlung der Bestrahlungssteuerdaten mit einer höheren der vorgegebenen Modifikationsstufen wiederholt, so dass eine geringere Beugungswirkung entsteht. Im Rahmen der Modifikation (Schritt S6a) können die Abstände und/oder die räumlichen Ausdehnungen der zu erzeugenden Photodisruptionsblasen verändert werden. Die Wiederholung erfolgt iterativ, bis in der Prognose der vorgegebene maximale Intensitätsschwellwert unterschritten wird.

Bei der Korrektur für eine optimierte Beugungswirkung werden die Bestrahlungssteuerdaten außerdem hinsichtlich der jeweiligen lokalen Strahlenergie und der Bestrahlungspunktabstände an die positionsabhängige Wechselwirkungseffizienz angepasst. Die Bestrahlungssteuerdaten können zweckmäßigerweise auch anhand des angepassten Modells der Augenlinse 2, das patientenindividuelle Eigenschaften der Augenlinse 2 repräsentiert, korrigiert werden . Das patientenangepasste Modell kann zu diesem Zweck in einem Zwischenschritt nach einer teilweisen Ermittlung der Bestrahlungssteuerdaten beispielsweise wie zu Fig. 2 beschrieben aktualisiert werden, um beispielsweise Lageänderungen des okulären Gewebes aufgrund von zwischenzeitlichen Augenbewegungen während der Behandlung zu berücksichtigen. Dies kann beispielsweise auch noch während der eigentlichen Bestrahlung wiederholt erfolgen. Hierzu wird der Abschwächer 15 vorübergehend wieder in den Beleuchtungsstrahlengang B geklappt. Zweckmäßigerweise werden dann die noch nicht in das okuläre Gewebe übertragenen Bestrahlungssteuerdaten anhand des aktualisierten Modells neu ermittelt.

Unmittelbar anschließend an die Erstellung der Bestrahlungssteuerdaten wird der eigentliche laserchirurgische Eingriff bei Therapie-Laserleistung anhand der Bestrahlungssteuerdaten durchgeführt (Schritt S7). Dabei werden beispielsweise durch die Laserstrahlung bei einer Pulsfrequenz von 100 kHz bis 1 MHz und einer Pulslänge von weniger als 1 ps, insbesondere von 300 fs, Photodisruptionsblasen (Kavitationsblasen) mit einer Pulsenergie von vorzugsweise maximal 0,5 µJ erzeugt. In der Folge kommt es zur Ausbildung von Defektstellen mit zwischenliegenden Gewebebrücken. Durch die beugungsoptimierten Bestrahlungssteuerdaten wird eine dreidimensionale Struktur in der Augenlinse erzeugt, die zu einer Auflockerung verhärteter Bereiche der Augenlinse führt, wodurch die Akkommodationsfähigkeit der Augenlinse verbessert wird. Dabei wird durch die beugungsoptimierte Anordnung der Defektstellen die konstruktive Interferenz infolge der Überlagerung aus Beugung an den Einzeldefekten vermieden.

Die Strahlenbelastung der Netzhaut durch Sekundärstrahlung kann dabei reduziert werden, indem die Therapie im hinteren Bereich der Augenlinse 2, beispielsweise mit der hintersten Inzision, begonnen wird, bevor im mittleren und vorderen Bereich der Augenlinse 2 weitere therapeutische Inzisionen erzeugt werden.

Durch den identischen Strahlengang für Analyse und Therapie ist das System 1 hinsichtlich der Betrachtungsposition selbstkalibrierend. Da die Bestrahlungssteuerdaten anhand der mit dem identischen Strahlengang ermittelten Informationen über Form/Struktur/Lage der Linse bestimmt werden, ist die Therapie mit hoher Genauigkeit möglich.

In einer alternativen Ausführungsform können zunächst aus dem/den Soll-Schnitt(en) auf herkömmliche Weise vorläufige Bestrahlungssteuerdaten mit in bekannter Weise äquidistant angeordneten Bestrahlungspunkten ermittelt werden. Aufgrund der Äquidistanz der Bestrahlungspunkte würden diese vorläufigen Bestrahlungssteuerdaten zu unerwünschter Störinterferenz führen, wenn sie unverändert in das okuläre Gewebe übertragen würden. Erfindungsgemäß werden die Bestrahlungssteuerdaten nachträglich beugungsoptimiert, indem die Abstände der Bestrahlungspunkte so modifiziert werden, so sie nicht-äquidistant angeordnet sind. Die Modifikation kann dabei wie oben beschrieben iterativ erfolgen.

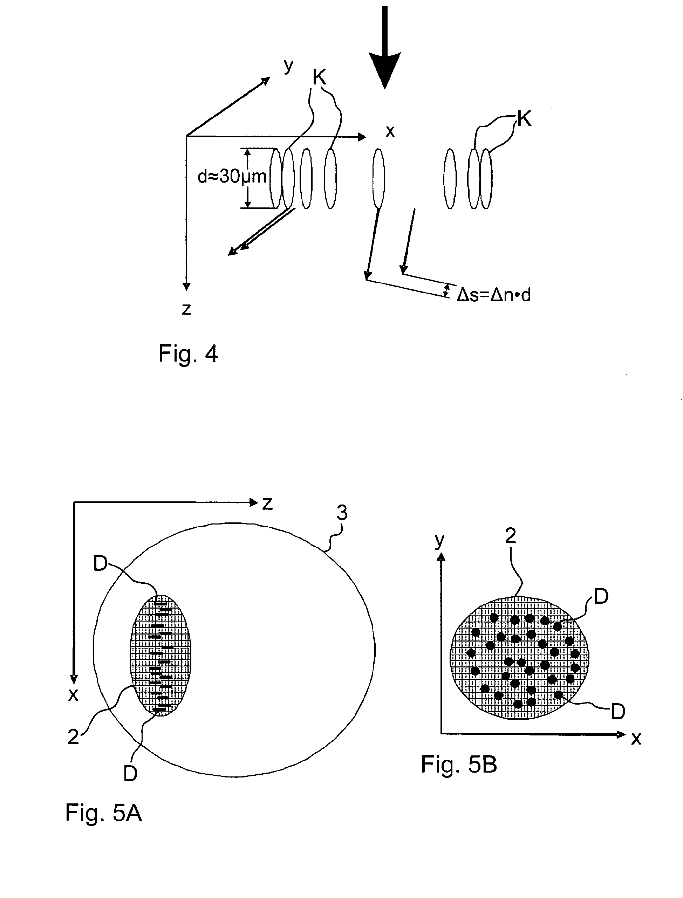

**Fig. 4** verdeutlicht das der Beugungsoptimierung zugrundeliegende Prinzip. Δn ist der Brechzahlunterschied zwischen dem okulären Gewebe und der Kavitätenflüssigkeit (Kammerwasser). Durch die nicht-äquidistant optimierten Abstände zwischen den an den in einer Ebene liegenden Bestrahlungspunkten entstandenen Kavitäten P wird unter den jeweiligen lokalen Beugungswinkeln (je enger der Abstand, desto größer der Beugungswinkel) ein gleichmäßiger Gangunterschied Δs vermieden und damit konstruktive Interferenz unterdrückt.

In den Fig. 5-10 sind beispielhafte Anordnungen von Kavitäten, Defektstellen beziehungsweise Schnittflächen, die mittels jeweiliger beugungsoptimierter Bestrahlungssteuerdaten zur Vermeidung von Interferenz in Augenlinsen 2 erzeugt werden können, in schematischer Schnittdarstellung dargestellt. Diese Anordnungen können als Grundmuster im obengenannten Sinne verwendet werden.

Zur Erzeugung der Defektstellen D gemäß der Anordnung in **Fig. 5** können die Bestrahlungspunkte beispielsweise auf einer durchgehenden Raumkurve angeordnet werden, die durch solche Bereiche der Augenlinse 2 verläuft, die zur Auflockerung einer Presbyopie geeignet sind. Dabei werden die Bestrahlungspunkte gerade so beabstandet, dass eine nicht-äquidistante Anordnung der Defektstellen D resultiert. Dies kann beispielsweise mittels eines Zufallsgenerators erfolgen, dessen Ausgaben zur Bestimmung der Abstände der Bestrahlungspunkte längs der Raumkurve verwendet wird. Alternativ kann eine zunächst äquidistante Anordnung von Bestrahlungspunkten, die zur Auflockerung einer presbyopen Linse geeignet ist, durch nachträgliches, zufälliges Verschieben (engl. "shuffle") der einzelnen Bestrahlungspunkte beugungsoptimiert werden, so dass Störinterferenz vermindert oder vermieden wird. Bei einer solchen Verschiebung wird zweckmäßigerweise eine maximale Verschiebungsweite vorgegeben.

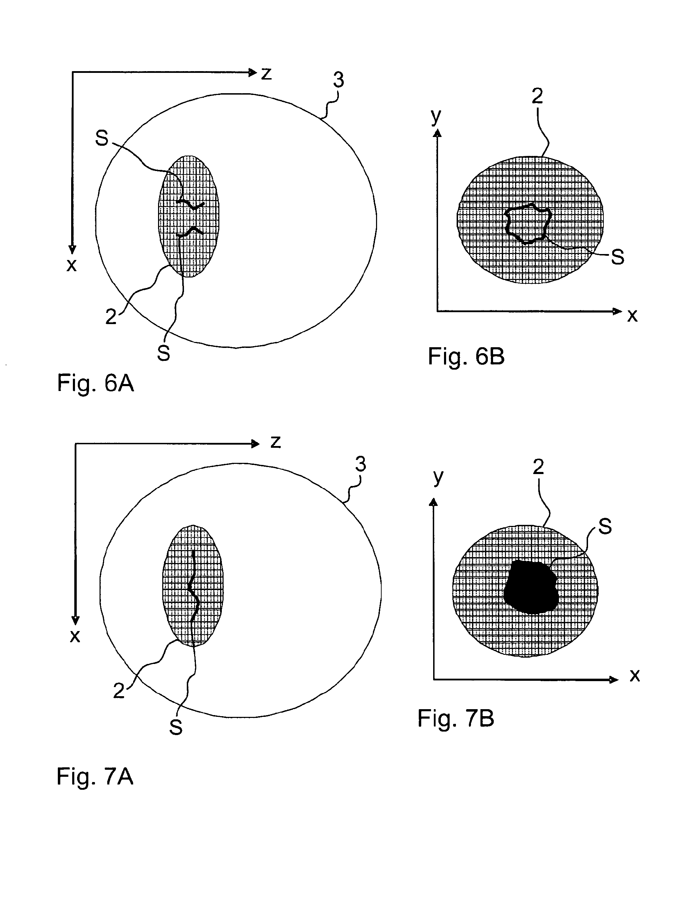

In der Ausführungsart gemäß **Fig. 6 und 7** wird die Laserstrahlung in der Augenlinse 2 derart appliziert, dass es zu einem Überlapp zwischen den einzelnen Kavitäten (nicht abgebildet) kommt. Aufgrund der geschlossenen Schnittführung wird die Ausbildung isolierter Defektstellen vermieden und dadurch die Beugung/Interferenz eingeschränkt. Durch den Verlauf und die Positionierung der photodisruptiven Schnitte werden gegeneinander verschiebbare Schnittflächen S erzeugt, wodurch die Akkommodationsfähigkeit der Augenlinse 2 verbessert wird. Durch die nicht-äquidistante Schnittführung in x-, y und z-Richtung wird die Wirkung der Struktur als diffraktiv wirkende Linse vermieden oder zumindest reduziert. In Fig. 6 sind die Schnitte S im wesentlichen parallel zur optischen Achse z angeordnet. In Fig. 7 sind die Schnitte S im wesentlichen senkrecht zur optischen Achse z angeordnet.

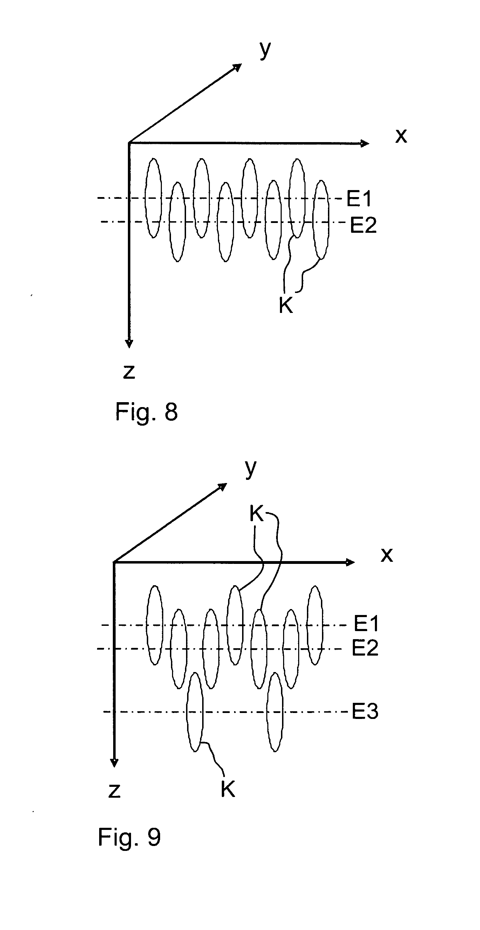

Eine nicht-äquidistante Anordnung von Kavitäten K gemäß **Fig. 8** hat den Vorteil, dass trotz der erfindungsgemäßen Unterdrückung von Interferenzen eine bezüglich der mechanischen Wirkung kontinuierliche Fläche von Photodisruptionen (Schnittfläche) entsteht. Die nicht-äquidistante Anordnung wird durch verschachtelte Anordnung zweier Ebenen E1, E2 von Bestrahlungspunkten mit jeweils äquidistanter Anordnung innerhalb der jeweiligen Ebene unter Versatz der Ebenen in z-Richtung erzielt. Die beiden Ebenen E1, E2 können beispielsweise identische Abstände zwischen den erzeugten Kavitäten K aufweisen.

Im Unterschied dazu sind die Abstände zwischen den Kavitäten K innerhalb der Ebenen in **Fig. 9** zwar äquidistant, unterscheiden sich aber zwischen den hier drei Ebenen E1, E2, E3. Die drei Ebenen sind mit zweifachem, unterschiedlichem Versatz in z-Richtung angeordnet.

Bei Grundmustern mit einer parametrisierbaren Anordnungsregel für die Bestrahlungspunkte wie in Fig. 8 und 9 gezeigt können Modifikationsstufen für eine stärkere Unterdrückung von Störinterferenz beispielsweise weitere Ebenen mit Bestrahlungspunkten umfassen. Zweckmäßigerweise weisen alle Ebenen stets paarweise unterschiedliche Abstände auf.

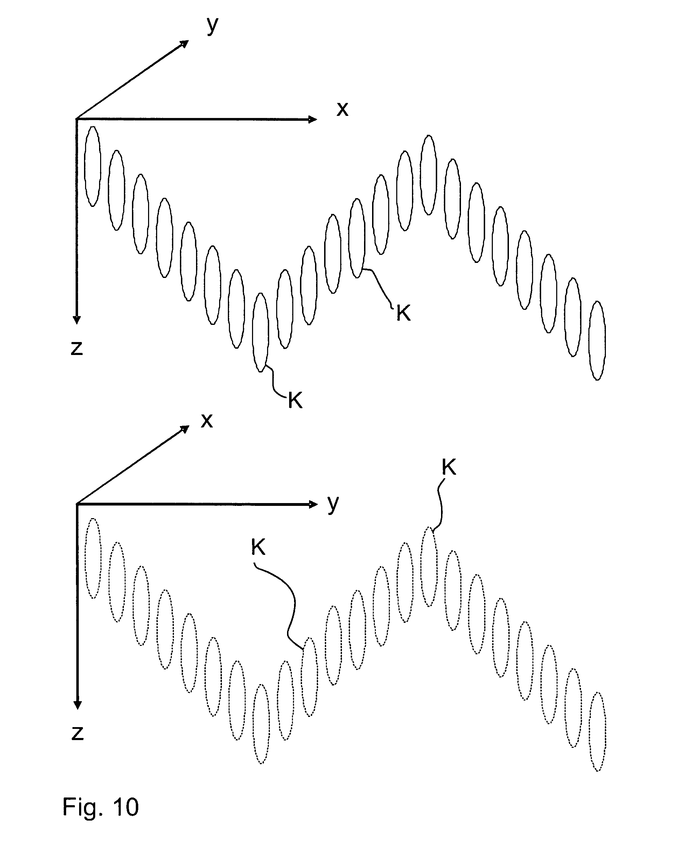

**Fig. 10** zeigt Kavitäten K in einer Augenlinse (nicht abgebildet), die mit einer in x-Richtung äquidistanten Anordnung mit mehrfachem, äquidistantem z-Versatz (oben) sowie mit wahlweise zusätzlichem mehrfachem äquidistantem z-Versatz (unten) vorliegen. Durch die insgesamt nicht-äquidistante Anordnung werden konstruktive Interferenzen unterdrückt.

In allen Ausführungsformen kann in Abhängigkeit der räumlichen Position der Abstand und/oder die applizierte Laserenergie zwischen den Fokuspositionen variiert werden, um angepasst an die Wechselwirkung mit dem zu behandelnden okulären Gewebe (Augenlinse, Cornea) optimale Photodisruptionen bei weitgehender Vermeidung von beugungsbedingten Effekten zu erreichen. Darüber hinaus kann in allen Ausführungsformen zusätzlich die innere Struktur des okulären Gewebes auf der Basis der Gewinnung von Patientendaten und gleichzeitig die Veränderung der Photodisruptionseffizienz in Abhängigkeit von der Position in x- y- und z-Richtung in der Berechnung des Bestrahlungssteuerdaten berücksichtigt werden.

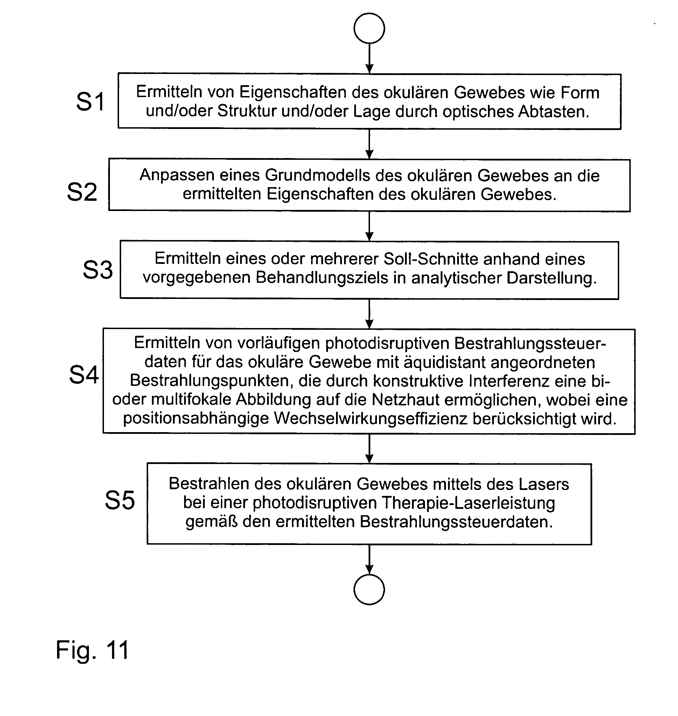

In **Fig. 11** ist ein Ablaufdiagramm für ein alternatives Behandlungs- und Betriebsverfahren dargestellt. Im Unterschied zu Fig. 3 werden jedoch im Ergebnis durch eine Femtosekunden-Lasertherapie der presbyopen menschlichen Augenlinse und/oder der Cornea durch Photodisruption aus Disruptionsblasen bestehende dreidimensionale und/oder zweidimensionale Strukturen mit einer refraktiven und/oder diffraktiven optischen Wirkung eingebracht, um refraktive Defizite des Auges zu kompensieren. Neben einer mechanischen Gewebeauflockerung können so zusätzliche optisch diffraktive oder refraktive Effekte erzeugt werden, welche dem Auge eine bi- oder multifokale Funktion ermöglichen und andererseits unerwünschte optisch diffraktive und/oder refraktive Effekte wie Störinterferenz gezielt unterdrückt.

Das Einbringen von Laserfleckstrukturen in okuläres Gewebe mit Hilfe eines fokussierten ultrakurzen Lasersystems bewirkt zunächst die Ausbildung von Disruptionsblasen, welche sich nach kurzer Zeit mit intraokulärer Flüssigkeit anfüllen. Dabei ist bekannt, dass im allgemeinen die Brechzahl der Flüssigkeit (Wasser) geringer ist als die des Gewebes, beispielsweise Kollagen.

Typische Brechzahlen im Auge sind:
Cornea: n=1,377
Kammerwasser: n=1,337
Glaskörper: n= 1,366
Linse: Gradientenlinsenstruktur mit n= 1,377 .... 1,404
(Jim Schwiegerling: "Visual and Ophthalmic Optics", SPIE Press Bellingham, Washington, USA, ISBN 0-8194-5629-2)

Wie aus den aufgeführten Daten ersichtlich, ist ein Brechzahlunterschied von 1,404-1,337=0,067 innerhalb des Linsenkerns und von 1,377-1,337= 0,04 innerhalb der Cornea zu erzielen, wenn dort lokal kammerwassergefüllte Kavitäten erzeugt werden. Werden Kavitäten mit einem Durchmesser in der Größenordnung von mindestens 10 µm erzeugt, können beispielsweise refraktive mikrooptische Strukturen entsprechend einer Fresnel-Linsenstruktur generiert werden. Sind die Durchmesser der Kavitäten geringer als 10 µm (geringer als 1 µm), also in der Größenordnung der Wellenlänge des visuellen Spektrums, können diffraktive Wirkungen entsprechend einer Fresnel-Zonenplatte erzielen, wenn mit regelmäßigen Bestrahlungspunktmustern entsprechende Kreisstrukturen erzeugt werden.

Auf diese Weise können zweidimensionale Phasengitter derart generiert werden, dass sie als diffraktive fokussierende oder defokussierende Linse wirken, um eine Sehschwäche zu kompensieren. Dies gelingt beispielsweise durch Anordnung derart, dass auf der Netzhaut eine Abbildung durch konstruktive Interferenz erzielt wird. Vorzugsweise wird eine bi- oder multifokale Abbildung erzeugt. Weiterhin können die Bestrahlungspunkte als nicht-äquidistante zweidimensionale Gitterstruktur ausgebildet werden, um mechanische Gleitebenen in das Gewebe einzubringen, dabei aber unerwünschte Beugungseffekte wie Störinterferenz zu unterdrücken. Darüber hinaus können dreidimensionale Phasengitter durch Einbringen mehrerer Bestrahlungspunktmuster in unterschiedlicher liegender Tiefe in Art einer Reihenschaltung einbringen. Damit lässt sich eine gewünschte diffraktive fokussierende oder defokussierende Wirkung verstärken, wenn insbesondere äquidistante Strukturen ausgewählt werden.

Es ist möglich, in einer Ausführungsform der Erfindung zwei- oder dreidimensionale refraktive und/oder diffraktive Linsenstrukturen mit einer beispielsweise um +3,5 dpt progressiven Nahaddition gegenüber der für ein scharfes Fernbild verantwortlichen Linsenbrechkraft zusätzlich in das Innere der Linse einzubringen, um bei presbyopen Augen das Lesen wieder zu ermöglichen.

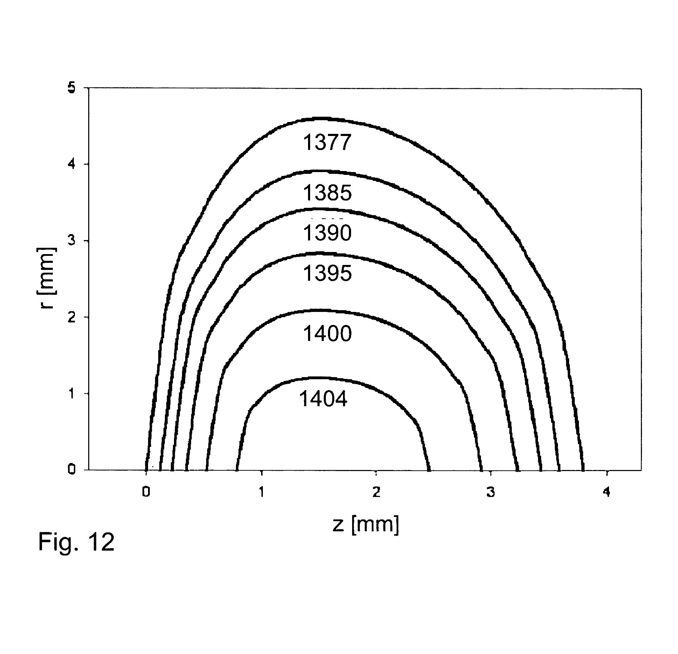

Bei der Ermittlung der Bestrahlungssteuerdaten wird zweckmäßigerweise die in **Fig. 12** schematisch dargestellte natürliche Gradientenlinsenstruktur des menschlichen Auges berücksichtigt (A. D. Priest, "The development of an average, anatomically based, young adult, GRIN eye model", Thesis (M.Sc.) University of Waterloo, 2004). Bei Einbringen einer zentralen zusätzlichen diffraktiven und/oder refraktiven Fresnel-Linsenstruktur innerhalb einer Tagespupille von etwa 2 mm im zentralen Kernbereich der Augenlinse kann zur Vereinfachung näherungsweise von einer homogenen Brechzahlverteilung ausgegangen werden. Bei größeren Pupillenöffnungen sind hingegen für eine hohe Genauigkeit die lokalen Brechzahlunterschiede zu den mit intraokulärer Flüssigkeit gefüllten Kavitäten im Rahmen einer dreidimensionalen Rechnung im einzelnen zu berücksichtigen, auch wenn die Bestrahlungspunkte im wesentlichen zweidimensional in einer zur optischen Achse oder einer zu auch der Sehachse des Auges senkrechten Ebene angeordnet werden.

### Bezugszeichenliste

- 1: Ophthalmologisches Lasersystem
- 2: Augenlinse
- 3: Auge
- 4: Laser
- 5: Strahlteiler
- 6: Scanoptik
- 7: Scannereinheit
- 8: Fokussieroptik
- 9: Optisches Phasenverzögerungssystem
- 10: Umlenkspiegel
- 11: Konfokale Aperturblende
- 12: Detektor
- 13: Verstärker
- 14: Steuereinheit
- 15: Abschwächer
- 16: Modulator
- 17: Fixiervorrichtung
- 18: Pulswähler

- B: Beleuchtungsstrahlengang
- C: Detektionsstrahlengang
- P: Abtastpunkt
- D: Defekt
- K: Kavität
- S: Schnittfläche
- E1/2/3: Ebenen von Bestrahlungspunkten

## Patentansprüche

1. Ophthalmologisches Lasersystem (1) zur photodisruptiven Bestrahlung einer Cornea, mit einem Ultrakurz-Puls-Laser (4), dessen Strahlung als Beleuchtungslicht über einen Beleuchtungsstrahlengang (B), der eine Scannereinheit (7) und eine Fokussieroptik (8) aufweist, in einem Untersuchungsbereich fokussierbar ist, und mit einer Steuereinheit (14), mittels derer folgende Schritte ausführbar sind:
- Ermitteln von Bestrahlungssteuerdaten für Photodisruptionen an mehreren Bestrahlungspunkten im Inneren der Cornea anhand mindestens eines vorgegebenen Soll-Schnittes derart, dass die Bestrahlungspunkte dreidimensional verteilt und nicht-äquidistant angeordnet sind, und
- Bestrahlen der Cornea gemäß den ermittelten Bestrahlungssteuerdaten, **gekennzeichnet dadurch, dass**:
mittels der Steuereinheit (14) zum Ermitteln der Bestrahlungssteuerdaten folgende Schritte ausführbar sind:
a) Ermitteln von vorläufigen Bestrahlungssteuerdaten gemäß dem mindestens einen Soll-Schnitt,
b) Prognostizieren eines aus den vorläufigen Bestrahlungssteuerdaten bei durchfallendem Licht resultierenden Interferenzmusters,
c) Ermitteln einer Intensität von Beugungslicht in der ersten oder einer höheren Ordnung des prognostizierten Interferenzmusters,
d) Vergleichen der ermittelten Intensität mit einem vorgegebenen Intensitätsschwellwert und
e) Wiederholen der Schritte a) bis d), wenn die ermittelte Intensität größer als der Intensitätsschwellwert ist, wobei in Schritt a) die vorläufigen Bestrahlungssteuerdaten modifiziert oder andere vorläufige Bestrahlungssteuerdaten ermittelt werden.

2. Ophthalmologisches Lasersystem (1) nach Anspruch 1, **dadurch gekennzeichnet, dass** der Soll-Schnitt für eine Erzeugung eines Flaps und/oder eines Lentikels ausgebildet ist.

3. Ophthalmologisches Lasersystem (1) nach Anspruch 1 oder 2, **dadurch gekennzeichnet, dass** die Steuereinheit (14) geeignet ist, die Bestrahlungspunkte regelmäßig anzuordnen.

4. Ophthalmologisches Lasersystem (1) nach Anspruch 3, wobei die Steuereinheit geeignet ist, die Bestrahlungspunkte regelmäßig anzuordnen, indem sie eine erste Teilmenge der Bestrahlungspunkte in einer ersten Ebene quer zu einer optischen Achse äquidistant anordnet und eine zweite Teilmenge der Bestrahlungspunkte in einer zweiten Ebene quer zu der optischen Achse äquidistant anordnet, wobei die zweite Ebene von der ersten Ebene um höchstens einen Durchmesser einer zu erzeugenden Photodisruption entfernt liegt.

5. Ophthalmologisches Lasersystem (1) nach Anspruch 3, **dadurch gekennzeichnet, dass** die Steuereinheit (14) geeignet ist, eine dritte Teilmenge der Bestrahlungspunkte in einer dritten Ebene quer zu der optischen Achse äquidistant anzuordnen, wobei sich der Abstand zu der ihre näherliegenden der beiden anderen Ebenen von dem Abstand zwischen den beiden anderen Ebenen unterscheidet.

6. Ophthalmologisches Lasersystem (1) nach einem der vorhergehenden Ansprüche, wobei eine nicht-äquidistante Anordnung der Bestrahlungspunkte mittels eines vor oder nach einer Laserverstärkungsanordnung angeordneten Pulswählers durch zeitlich nicht-äquidistante Steuersignale erzeugbar ist.

7. Ophthalmologisches Lasersystem (1) nach einem der vorhergehenden Ansprüche, **gekennzeichnet durch** eine Anordnung zur konfokalen Detektion von Strahlung aus dem Untersuchungsbereich.

8. Ophthalmologisches Lasersystem (1) zur photodisruptiven Bestrahlung okulären Gewebes, mit einem Laser (4), dessen Strahlung als Beleuchtungslicht über einen Beleuchtungsstrahlengang (B), der eine Scannereinheit (7) und eine Fokussieroptik (8) aufweist, in einem Untersuchungsbereich fokussierbar ist, **gekennzeichnet durch** eine Steuereinheit (14), mittels derer folgende Schritte ausführbar sind:
- Ermitteln von Bestrahlungssteuerdaten für Photodisruptionen an mehreren Bestrahlungspunkten im Inneren des okulären Gewebes anhand mindestens eines vorgegebenen Soll-Schnittes derart, dass die Bestrahlungspunkte regelmäßig angeordnet sind und **durch** konstruktive Interferenz in **durch** das okuläre Gewebe hindurchtretendem Licht eine Kompensation eines refraktiven Defizits resultiert, und
- Bestrahlen des okulären Gewebes gemäß den ermittelten Bestrahlungssteuerdaten.

9. Ophthalmologisches Lasersystem (1) nach einem der vorhergehenden Ansprüche, wobei vor dem Ermitteln von Bestrahlungssteuerdaten mindestens eine Eigenschaft des okulären Gewebes ermittelt und bei der Ermittlung der Bestrahlungssteuerdaten und/oder bei einer Korrektur der Bestrahlungssteuerdaten berücksichtigt wird.

10. Ophthalmologisches Lasersystem (1) nach einem der vorhergehenden Ansprüche, wobei bei der Ermittlung der Bestrahlungssteuerdaten und/oder bei einer Korrektur der Bestrahlungssteuerdaten eine bezüglich des okulären Gewebes positionsabhängige Effizienz der photodisruptiven Bestrahlung berücksichtigt wird.

## Claims

1. Ophthalmic laser system (1) for photo-disruptive irradiation of a cornea, comprising an ultrashort-pulse laser (4), the radiation of which is focusable in an examination region as illumination light via an illumination beam path (B), which includes a scanner unit (7) and focusing optics (8), and comprising a control unit (14), by means of which the following steps are executable:
- establishing irradiation control data for photo-disruptions at a plurality of irradiation points in the interior of the cornea on the basis of at least one predetermined intended cut in such a way that the irradiation points are distributed in three dimensions and not arranged equidistantly, and
- irradiating the cornea in accordance with the established irradiation control data, **characterized in that**:
by means of the control unit (14) for establishing the irradiation control data, the following steps are executable:
a) establishing preliminary irradiation control data in accordance with the at least one intended cut,
b) predicting a resulting interference pattern from the preliminary irradiation control data in the case of passing-through light,
c) establishing an intensity of first or higher order diffraction light of the predicted interference pattern,
d) comparing the established intensity with a predetermined intensity threshold and
e) repeating steps a) to d) if the established intensity is greater than the intensity threshold, wherein the preliminary irradiation control data are modified, or other preliminary irradiation control data are established, in step a).

2. Ophthalmic laser system (1) according to Claim 1, **characterized in that** the intended cut is configured for producing a flap and/or a lenticule.

3. Ophthalmic laser system (1) according to Claim 1 or 2, **characterized in that** the control unit (14) is suitable for arranging the irradiation points in a regular manner.

4. Ophthalmic laser system (1) according to Claim 3, wherein the control unit is suitable for arranging the irradiation points in a regular manner by virtue of arranging a first portion of the irradiation points equidistantly in a first plane perpendicular to an optical axis and arranging a second portion of the irradiation points equidistantly in a second plane perpendicular to the optical axis, wherein the distance from the second plane to the first plane is no more than one diameter of a photo-disruption to be generated.

5. Ophthalmic laser system (1) according to Claim 3, **characterized in that** the control unit (14) is suitable for arranging a third portion of the irradiation points equidistantly in a third plane perpendicular to the optical axis, wherein the distance to the closer one of the two other planes does not equal the distance between the two other planes.

6. Ophthalmic laser system (1) according to one of the preceding claims, wherein a non-equidistant arrangement of the irradiation points is generable by control signals that are not equidistant in time by means of a pulse selector arranged upstream or downstream of a laser amplification arrangement.

7. Ophthalmic laser system (1) according to one of the preceding claims, **characterized by** an arrangement for confocal detection of radiation from the examination region.

8. Ophthalmic laser system (1) for photo-disruptive irradiation of ocular tissue, comprising a laser (4), the radiation of which is focusable in an examination region as illumination light via an illumination beam path (B), which includes a scanner unit (7) and focusing optics (8), **characterized by** a control unit (14), by means of which the following steps are executable:
- establishing irradiation control data for photo-disruptions at a plurality of irradiation points in the interior of the ocular tissue on the basis of at least one predetermined intended cut in such a way that the irradiation points are arranged in a regular manner and a compensation of a refractive deficit results from constructive interference in the light passing through the ocular tissue, and
- irradiating the ocular tissue by means of the established irradiation control data.

9. Ophthalmic laser system (1) according to one of the preceding claims, wherein, prior to establishing irradiation control data, at least one property of the ocular tissue is established and taken into account when establishing the irradiation control data and/or when correcting the irradiation control data.

10. Ophthalmic laser system (1) according to one of the preceding claims, wherein an efficiency of the photo-disruptive irradiation, which is dependent on position in respect of the ocular tissue, is taken into account when establishing the irradiation control data and/or when correcting the irradiation control data.

## Revendications

1. Système à laser ophtalmologique (1) destiné à l'irradiation de photocautérisation d'une cornée, comprenant un laser à impulsions ultra-courtes (4) dont le rayonnement peut être concentré dans une zone d'examen sous la forme d'une lumière d'éclairage par le biais d'un trajet de rayon d'éclairage (B), lequel présente une unité de balayage (7) et une optique de concentration (8), et comprenant une unité de commande (14) à l'aide de laquelle les étapes suivantes peuvent être exécutées :
- détermination de données de commande d'irradiation pour des photocautérisations en plusieurs points d'irradiation à l'intérieur de la cornée à l'aide d'au moins une coupe voulue prédéfinie, de telle sorte que les points d'irradiation sont distribués de manière tridimensionnelle et ne sont pas disposés de manière équidistante, et
- irradiation de la cornée conformément aux données de commande d'irradiation déterminées,
**caractérisé en ce que** :
au moyen de l'unité de commande (14) en vue de déterminer les données de commande d'irradiation, les étapes suivantes peuvent être exécutées :
a) détermination de données de commande d'irradiation provisoires selon l'au moins une coupe voulue,
b) prédiction d'un modèle d'interférence résultant des données de commande d'irradiation provisoires en présence de lumière transmise,
c) détermination d'une intensité de la lumière de diffraction dans le premier ordre ou un ordre supérieur du modèle d'interférence prédit,
d) comparaison de l'intensité déterminée avec un seuil d'intensité prédéfini et
e) répétition des étapes a) à d) lorsque l'intensité déterminée est supérieure au seuil d'intensité, les données de commande d'irradiation provisoires étant modifiées à l'étape a) ou d'autres données de commande d'irradiation provisoires étant déterminées.

2. Système à laser ophtalmologique (1) selon la revendication 1, **caractérisé en ce que** la coupe voulue est conçue pour la production d'un rabat et/ou d'une lenticule.

3. Système à laser ophtalmologique (1) selon la revendication 1 ou 2, **caractérisé en ce que** l'unité de commande (14) est conçue pour réaliser une disposition régulière des points d'irradiation.

4. Système à laser ophtalmologique (1) selon la revendication 3, avec lequel l'unité de commande est conçue pour réaliser une disposition régulière des points d'irradiation en ce qu'elle dispose une première quantité partielle de points d'irradiation de manière équidistante dans un premier plan transversal par rapport à un axe optique et dispose une deuxième quantité partielle de points d'irradiation de manière équidistante dans un deuxième plan transversal par rapport à l'axe optique, le deuxième plan étant espacé du premier plan au maximum d'un diamètre d'une photocautérisation à produire.

5. Système à laser ophtalmologique (1) selon la revendication 3, **caractérisé en ce que** l'unité de commande (14) est conçue pour disposer une troisième quantité partielle de points d'irradiation de manière équidistante dans un troisième plan transversal par rapport à l'axe optique, l'écart par rapport à celui des deux autres plans qui lui est le plus proche étant différent de l'écart entre les deux autres plans.

6. Système à laser ophtalmologique (1) selon l'une des revendications précédentes, avec lequel un arrangement non équidistant des points d'irradiation peut être produit par des signaux de commande non équidistants dans le temps au moyen d'un sélecteur d'impulsion disposé avant ou après un arrangement d'amplification de laser.

7. Système à laser ophtalmologique (1) selon l'une des revendications précédentes, **caractérisé par** un arrangement destiné à la détection confocale du rayonnement issu de la zone d'examen.

8. Système à laser ophtalmologique (1) destiné à l'irradiation de photocautérisation du tissu oculaire, comprenant un laser (4) dont le rayonnement peut être concentré dans une zone d'examen sous la forme d'une lumière d'éclairage par le biais d'un trajet de rayon d'éclairage (B), lequel présente une unité de balayage (7) et une optique de concentration (8), **caractérisé par** une unité de commande (14) à l'aide de laquelle les étapes suivantes peuvent être exécutées :
- détermination de données de commande d'irradiation pour des photocautérisations en plusieurs points d'irradiation à l'intérieur du tissu oculaire à l'aide d'au moins une coupe voulue prédéfinie, de telle sorte que les points d'irradiation sont disposés de manière régulière et qu'une compensation d'un déficit réfractif résulte d'une interférence structurale dans la lumière qui traverse le tissu oculaire, et
- irradiation du tissu oculaire conformément aux données de commande d'irradiation déterminées.

9. Système à laser ophtalmologique (1) selon l'une des revendications précédentes, avec lequel au moins une propriété du tissu oculaire est déterminée avant la détermination de données de commandes d'irradiation et prise en compte lors de la détermination des données de commande d'irradiation et/ou lors d'une correction des données de commande d'irradiation.

10. Système à laser ophtalmologique (1) selon l'une des revendications précédentes, avec lequel une efficacité de l'irradiation de photocautérisation, dépendant de la position par rapport au tissu oculaire, est prise en compte lors de la détermination des données de commande d'irradiation et/ou lors d'une correction des données de commande d'irradiation.
